# EUROPÄISCHE PATENTSCHRIFT

(11) **EP 0 345 591 B1**
(45) Veröffentlichungstag und Bekanntmachung des Hinweises auf die Patenterteilung: **31.03.1993**
(21) Anmeldenummer: 89109675.2
(22) Anmeldetag: 30.05.1989
(51) Int. Cl.: C07C 217/54, C07C 215/48, C07D 333/20, C07D 213/36, A61K 31/135, A61K 31/215, A61K 31/19, A61K 31/38, A61K 31/44, A23K 1/16

(54) **Propanolaminderivate**
Propanol amine derivatives
Dérivés de la propanolamine

(30) Priorität: 10.06.1988 CH 2245/88
(43) Veröffentlichungstag der Anmeldung: 13.12.1989
(73) Patentinhaber: F. HOFFMANN-LA ROCHE AG, 4002 Basel (CH)
(72) Erfinder: Kienzle, Frank, Dr., CH-4113 Flüh (CH)
(74) Vertreter: Lederer, Franz, Dr.

(56) Entgegenhaltungen:
- EP-A- 0 007 204
- US-A- 3 804 899
- US-A- 4 622 342

## Beschreibung

Die vorliegende Erfindung betrifft neue Propanolaminderivate als solche sowie als therapeutische Wirkstoffe, insbesondere zur Behandlung der Fettsucht, des Diabetes mellitus und von Zuständen, die mit erhöhtem Proteinabbau verbunden sind; ferner pharmazeutische Präparate und Futtermittel für Masttiere, enthaltend ein solches Propanolaminderivat, Verfahren zur Herstellung dieser Derivate bzw. dieser Präparate und Futtermittel, sowie die Verwendung der besagten Propanolaminderivate zur Herstellung der obigen pharmazeutischen Präparate.

Die Propanolaminderivate der vorliegenden Erfindung sind Verbindungen der Formel 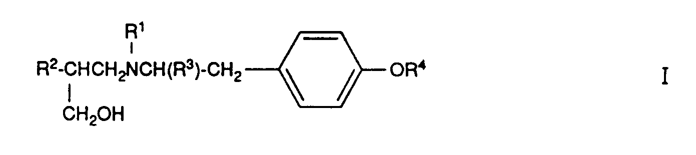
worin
- R¹: Wasserstoff oder eine Gruppe der Formel 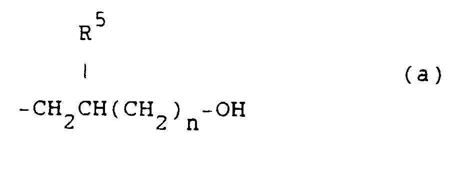
- n: die Zahl 0 oder 1
- R² und R⁵: Phenyl, m-Halophenyl, m-Trifluormethylphenyl, Thienyl oder Pyridyl
- R³: Wasserstoff oder Methyl
- R⁴: Wasserstoff, -CH₂COOH, -CH₂COO-C₁₋₄-Alkyl, -(CH₂)₂O-C₁₋₄-Alkyl oder -(CH₂)₂O(CH₂)₁₋₄-C₆H₅ ist,
und physiologisch verträgliche Salze davon.

An Stelle einer Propanolgruppierung enthalten die Verbindungen in der US-Patentschrift 4 622 342 eine Aethanolgruppierung.

Unter den m-Halophenylgruppen R² oder R⁵ ist m-Chlorphenyl bevorzugt, unter den Thienyl- und Pyridylgruppen 2-Thienyl bzw. 2-Pyridyl. C₁₋₄-Alkyl bezeichnet geradkettige oder verzweigte Reste mit 1-4 C-Atomen, wie Methyl, Aethyl, Propyl, Isopropyl, Butyl und Isobutyl.

Die Verbindungen der Formel I bilden mit Säuren Salze, die ebenfalls Gegenstand der Erfindung sind. Beispiele solcher Salze sind Salze mit physiologisch verträglichen Mineralsäuren, wie Salzsäure, Bromwasserstoffsäure, Schwefelsäure, Phosphorsäure; oder mit organischen Säuren, wie Oxalsäure, Methansulfonsäure, Essigsäure, Propionsäure, Zitronensäure, Maleinsäure, Bernsteinsäure, Aepfelsäure, Fumarsäure, Phenylessigsäure oder Salicylsäure. Carbonsäuren der Formel I können als Salze vorliegen. Beispiele für solche Salze sind Alkalimetall-, Erdalkalimetall-, Ammonium- und Aethanolammoniumsalze.

Die erfindungsgemässen Verbindungen enthalten zumindest ein asymmetrisches Kohlenstoffatom und können somit als optisch aktive Enantiomere, als Diastereomere oder als Racemate vorliegen.

Unter des Verbindungen der Formel I sind diejenigen bevorzugt, worin R¹ Wasserstoff, R² Phenyl, m-Halophenyl, insbesondere m-Chlorphenyl, oder m-Trifluormethylphenyl, Thienyl oder Pyridyl, insbesondere 2- oder 3-Pyridyl, R⁴ Wasserstoff, 2-Aethoxyäthyl, 2-Phenäthoxyäthyl oder Aethoxycarbonylmethyl ist, insbesondere diejenigen, worin das an einer allfälligen Methylgruppe R³ gebundene C-Atom die R-Konfiguration hat. Besonders bevorzugt sind solche Verbindungen, worin R² Phenyl oder 2-Thienyl und R⁴ 2-Aethoxyäthyl oder Aethoxycarbonylmethyl ist. Beispiele solcher Verbindungen sind die folgenden
(R oder S)-β-[[[p-(2-Aethoxyäthoxy)phenäthyl]amino]methyl]phenäthyl-alkohol,
[p-[2-[[β-(Hydroxymethyl)phenäthyl]amino]äthyl]phenoxy]essigsäureäthylester,
β-[[[(R)-p-(2-Aethoxyäthoxy)-α-methylphenäthyl]amino]methyl]phenäthylalkohol und
β-[[[p-(2-Aethoxyäthoxy)phenäthyl]amino]methyl]-2-thiophenäthanol.

Bevorzugt sind ferner die Verbindungen der Formel I, worin R¹ eine Gruppe der Formel (a) darstellt, in der entweder n die Zahl 1 und R⁵ Phenyl oder m-Halophenyl, insbesondere m-Chlorphenyl, oder n die Zahl 0 und R⁵ Phenyl ist, insbesondere worin das an der Gruppe R⁵ gebundene C-Atom die R-Konfiguration hat, R² Phenyl oder m-Halophenyl, insbesondere m-Chlorphenyl, R³ Wasserstoff und R⁴ 2-Aethoxyäthyl ist, z.B. der
(RS)-m-Chlor-β-[p-(2-äthoxyäthoxy)phenäthyl][[[(R)-β-hydroxyphenäthyl]amino]methyl]phenäthylalkohol.

Die Produkte der vorliegenden Erfindung können in an sich bekannter Weise dadurch hergestellt werden, dass man
a) ein Amid der Formel 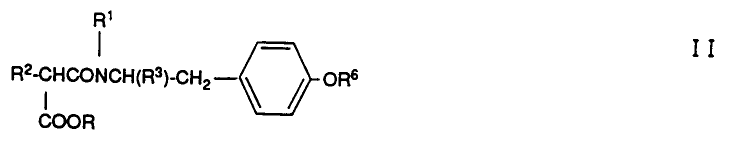 oder 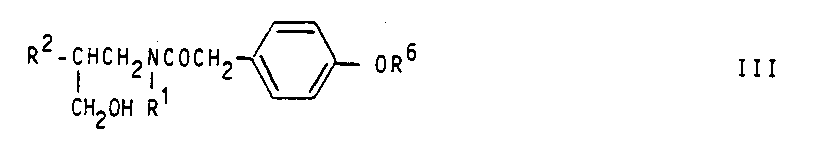 worin R C₁₋₄-Alkyl bedeutet und
   worin R¹, R² oder R³ die obige Bedeutung haben und
   R⁶ Wasserstoff, -(CH₂)₂O-C₁₋₄-Alkyl oder -(CH₂)₂O(CH₂)₁₋₄-C₆H₅ ist,
   reduziert und
b) gewünschtenfalls ein Phenol der Formel I, worin R⁴ Wasserstoff ist, zu einer Verbindung der Formel I, worin R⁴ eine Gruppe -CH₂-COOH oder -CH₂COO-C₁₋₄-Alkyl ist, veräthert und
c) gewünschtenfalls eine Verbindung der Formel I in ein Salz überführt.

Die Reduktion nach Verfahrensvariante a) kann man zweckmässig mittels eines komplexen Metallhydrids, wie Lithiumaluminiumhydrid (LiAlH₄), in einem Lösungsmittel, wie einem Aether, z.B. Diäthyläther, Monoglyme, Diglyme oder Tetrahydrofuran (THF) durchführen, bei einer Temperatur bis zur Rückflusstemperatur des Reaktionsgemisches, vorzugsweise bei Raumtemperatur.

Die Verätherung nach der Variante b) kann man mit einer Verbindung der Formel X-R⁴⁰ bewerkstelligen, worin R⁴⁰ eine Gruppe -CH₂COOH oder -CH₂COO-C₁₋₄-Alkyl und X Halogen, zweckmässig Jod, oder eine Sulfonatgruppe, z.B. Methansulfonat, ist. Die Reaktion wird zweckmässig in einem Lösungsmittel, wie einem Keton, z.B. Aceton, einem Aether, wie THF, oder in Dimethylformamid, in Gegenwart einer Base, wie einem Alkalimetallhydroxyd, z.B. Kalium- oder Natriumhydroxyd bei einer Temperatur bis zu Rückflusstemperatur, vorzugsweise bei Raumtemperatur, durchgeführt.

Die Amide der Formel II und III kann man in an sich bekannter Weise herstellen.

So entstehen die Amide II durch Reaktion eines Amins der Formel 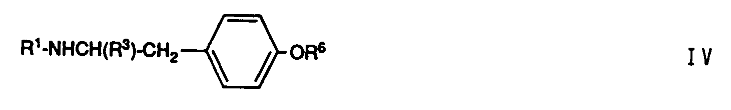
mit einem Diester der Formel

R²-CH(COOR)₂ V

worin R C₁₋₄-Alkyl ist.
Amide der Formel II, worin R¹ eine Gruppe (a) ist, kann man auch erhalten durch Reaktion eines sekundären Amins der Formel I, worin R¹ Wasserstoff ist, mit einem Diester V.

Diese Reaktionen kann man in einem Lösungsmittel, wie einem Aether, z.B. Diglyme, bei Temperaturen bis zur Rückflusstemperatur, zweckmässig bei etwa 90-100°C, durchführen. Zur Herstellung des Ausgangsamides des nachstehenden Beispiels 1 wurde auf diese Weise eine Lösung von 50 g Phenylmalonsäurediäthylester in 50 mg Diglyme mit 25 g p-(2-Aethoxyaethoxy)phenäthylamin versetzt und 48 Std. bei 95°C gerührt. Nach Abkühlung wurde das überschüssige Lösungsmittel abgedampft. Der Rückstand wurde an Kieselgel mit Essigester/Hexan (1:2) chromatographiert. Man erhielt den [[[p-(2-Aethoxyäthoxy)phenäthyl]carbamoyl]phenyl]essigsäureäthylester, Smp. 80°C. Die Ausgangsamide der Beispiele 2.a), 2.l) bzw. 2.o) wurden ebenfalls kristallin (Smp. 108°C, 94-95°C bzw. 110-111°C) erhalten.

Die Amide III entstehen durch Reaktion eines Alkohols der Formel 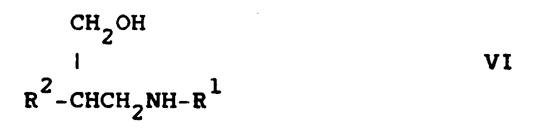
mit einer Verbindung der Formel 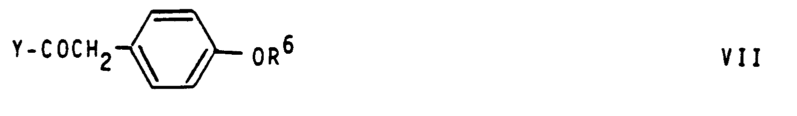
worin Y Halogen, insbesondere Chlor, oder nieder-Alkoxy ist.
Diese Reaktion lässt sich auf der gleichen Weise bewerkstelligen wie die weiter oben beschriebene Reaktion eines Amins IV mit einem Diester V. Zur Herstellung der Ausgangsamide III der Beispiele 3.a) und 3.b) wurden 50 g p-(2-Aethoxyaethoxy)phenylessigsäureäthylester in 50 ml Diglyme langsam mit 25 g (R oder S)-β-(Aminomethyl)phenäthylalkohol versetzt und 48 Std. bei 95°C gerührt. Dann wurde abgekühlt und eingedampft und der Rückstand wurde an Kieselgel mit Essigester/Hexan (1:2) chromatographiert.

Die Alkohole VI lassen sich durch Reduktion der entsprechenden Säuren der Formel 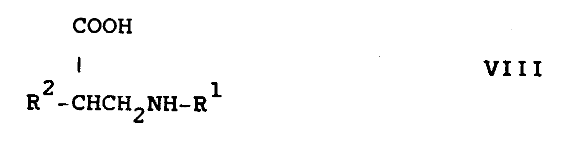
herstellen. Diese Reduktion kann man analog der weiter oben beschriebenen Reduktion eines Amids II oder III bewerkstelligen.

Ein Racemat der Formel VI lässt sich durch Salzbildung mit einer optisch aktiven Säure, wie L- oder D-Dibenzoylweinsäure, leicht aufspalten und eignet sich daher besonders als Zwischenprodukt bei der Herstellung von optisch aktiven Verbindungen III und I.

Zur Herstellung der Ausgangsalkohole VI der Beispiele 3.a) und 3.b) wurden 10 g 3-Amino-2-phenylpropancarbonsäure in 150 ml Monoglyme mit 3 g LiAlH₄ 20 Std. gerührt. Dann wurden langsam 12 ml Wasser und 3 ml 10%-ige Natronlauge dazugegeben. Das Gemisch wurde filtriert und das Filtrat wurde nochmals mit Toluol eingedampft. Man erhielt 8,4 g β-(Aminomethyl)phenäthylalkohol.

Zur Herstellung der R- und S-Enantiomeren dieses Alkohols wurde dieser in 200 ml THF gelöst und mit einer Lösung von Dibenzoyl-L-weinsäure versetzt. Nach einem Tag wurden Kristalle abfiltriert und bis zu konstanten Drehungen aus Aethanol umkristallisiert. Man erhielt das (R oder S)-β-(Aminomethyl)phenäthylalkohol-(S,S)-2, 3-di-O-benzoyltartrat, Smp. 172 173°C, [α]_{D} -28,4° (DMSO, c= 1%).

Die Mutterlaugen wurden mit einem stark basischen Ionenaustauscher behandelt, filtriert und eingedampft. Der ölige Rückstand des freien mit dem Enantiomeren angereicherten Amins wurde wie oben beschrieben, jedoch diesmal mit Dibenzoyl-D-weinsäure behandelt. Man erhielt das (R oder S)-β-(Aminomethyl)phenäthylalkohol-(R,R)-2, 3-di-O-benzoyltartrat, Smp. 172 - 173°C, [α]_{D} +28,2° (DMSO, c= 1%).

Die Verbindungen IV, V, VII und VIII kann man, soweit sie nicht bekannt sind, in an sich bekannter Weise z.B. in Analogie zu bekannten Verbindungen, herstellen. So sind die Diester V nach der in Organic Synthesis, Collective Vol. 2, Edited by A.H. Blatt (1943) 288-289 beschriebenen Methode aus den entsprechenden Essigsäureestern erhältlich.

Die erfindungsgemässen Propanolaminderivate können als Wirkstoffe in pharmazeutischen Präparaten zur Behandlung von Fettsucht und/oder des Diabetes mellitus, insbesondere des obesen erwachsenen Diabetikers verwendet werden. Im Tierexperiment wurde auf Verabreichung der besagten Derivate ein gesteigerter Katabolismus, vor allem der Fette beobachtet. Weiterhin wurde beobachtet, dass die erfindungsgemässen Propanolaminderivate die Bildung von braunem Fettgewebe bei Ratten und obes-hyperglykämischen Mäusen stimulieren. Bekanntlich wird Defekten des braunen Fettgewebes eine wesentliche Rolle bei der Entstehung der Fettsucht zugeschrieben. An obes-hyperglykämischen Mäusen und an Streptozotocin-diabetischen Ratten haben diese Derivate einen ausgeprägten antidiabetischen Effekt, indem sie hypoglykämisch wirken und die Glykosurie vermindern. Die erfindungsgemässen Propanolaminderivate zeigen nur eine sehr geringe Wirkung auf Herztätigkeit und Kreislauf. Zudem haben sie nur eine schwache Wirkung auf die Insulin-Ausschüttung. Dies ist von Bedeutung, da bekanntlich erhöhtem Plasma-Insulin eine wichtige Rolle bei der Pathogenese der Fettsucht beigemessen wird. Die Dosierung kann in Abhängigkeit von der Wirkungsstärke der einzelnen Verbindungen und den individuellen Bedürfnissen des Patienten 0,5-1000 mg, vorzugsweise 2-200 mg pro Tag für einen Erwachsenen betragen, wobei die Dosis als Einzeldosis oder in mehreren Dosen über den Tag verabreicht werden kann.

Ferner konnte mit den erfindungsgemässen Propanolaminderivaten im Tierexperiment eine Erhöhung des Proteingehaltes und eine Erniedrigung des Fettgehalts im Körper nachgewiesen werden. Die besagten Derivate führen demnach zu einer Erhöhung der mageren Körpermasse auf Kosten des Fettanteils. Daher können sie in der Humanmedizin zur Behandlung von Zuständen, die mit erhöhtem Proteinabbau verbunden sind, z.B. bei Rekonvaleszenz nach Operationen, verwendet werden. Dabei sind die Verabreichungsdosen die gleichen wie bei der Behandlung der Fettsucht und/oder des Diabetes mellitus.

Die erfindungsgemässen Propanolaminderivate können auch in der Ernährung von Masttieren, wie Rindern, Schweinen, Schafen und Geflügel, Verwendung finden. Dabei können die Verabreichungsformen die gleichen sein wie für Vitamine. Die besagten Derivate können auch als Futterzusatz in Dosen von 0,01-100 mg/kg je nach Substanz, Tierart und Alter eingesetzt werden.

Die pharmazeutischen Präparate enthalten den Wirkstoff zusammen mit einem verträglichen pharmazeutischen, organischen oder anorganischen Trägermaterial, wie Wasser, Gelatine, Gummi arabicum, Lactose, Stärke, Magnesiumstearat, Talk, pflanzliche Oele, Polyalkylenglykole oder Vaseline. Die pharmazeutischen Präparate werden vorzugsweise oral, z.B. in Form von Tabletten, Kapseln, Pillen, Pulver, Granulaten, Lösungen, Sirupen, Suspensionen oder Elixiren verabreicht. Die Verabreichung kann aber auch parenteral, z.B. in Form von sterilen Lösungen, Suspensionen oder Emulsionen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität der neuen Verbindungen der Formel I wird aus den nachstehenden Versuchsresultaten deutlich:

### 1) Wirkung auf den Sauerstoffverbrauch

Männliche Albinoratten im Gewicht von 160-180 g wurden nach 24 Stunden Fasten in Stoffwechselkäfige gesetzt. Die Käfige wurden mit konstant 6 Liter Raumluft/Minute, die bei einem Taupunkt von 11°C äquilibriert wurde, belüftet. Von der Abluft wurde nach erneuter Aequilibrierung während Perioden von jeweils 14 Minuten Proben gesammelt und der Sauerstoff- und CO₂-Gehalt analysiert. Nach einer Anpassungszeit von 4 Stunden erhielten die in Gruppen zu 6 aufgeteilten Tiere entweder Placebo (5% Gummi arabicum) oder die Testsubstanz (suspendiert in 5% Gummi arabicum) per os. Danach wurden 12 Stunden lang die Bestimmungen durchgeführt. In Tabelle I ist der Prozentsatz des gemittelten Sauerstoffverbrauchs nach Medikation während der ersten 3 Stunden und der gesamten Versuchsdauer (12 Stunden) vom Sauerstoffverbrauch der Anpassungsperiode angegeben, wobei entsprechende Korrekturen für Aenderungen in der Placebo-Gruppe berücksichtigt wurden.

**Tabelle I**

| Verbindung von Beispiel No. | Dosis mg/kg | Dosis µM/kg | O₂-Verbrauch % vom Wert der Vorperiode | |
|---|---|---|---|---|
| | | | 1.-3. Stunde | 1.-12. Stunde |
| 2.a) | 4,5 | 10 | 149 | 113 |
| 2.b) | 1,5 | 3 | 124 | 107 |
| 2.c) | 1,5 | 3 | 132 | 108 |
| 2.d) | 1,8 | 3 | 152 | 127 |
| 2.j) | 5,1 | 10 | 137 | 113 |
| 2.o) | 4,4 | 10 | 147 | 115 |
| 3.a) | 1,3 | 3 | 152 | 112 |
| 4 | 1,2 | 3 | 145 | 112 |

### 2) Wirkung auf die Herzfrequenz

Männliche Albino-Ratten im Gewicht von 250-320 g erhielten 6 Stunden nach Futterentzug entweder Placebo (5% Gummi arabicum) oder die Testsubstanz (suspendiert in 5% Gummi arabicum) per os. Die Tiere wurden anschliessend in perforierten Plastik-Röhren immobilisiert. Mittels bipolarer subkutaner Nadelelektroden wurde das Elektrokardiogramm abgeleitet. Die R-Zacke wurde zur Aktivierung eines Schlag-Zählers verwendet. Die 1 und 3 Stunde nach Beendigung gemessene Herzfrequenz ist in Tabelle II als Prozentsatz der Kontrollwerte (Placebo) angegeben.

**Tabelle II**

| Verbindung von Beispiel | Dosis (mg/kg) | Herzfrequenz (% von Kontrollen) | |
|---|---|---|---|
| | | 1 Stunde | 3 Stunde |
| 2a | 30 | 105 | 100 |
| | 100 | 121 | 112 |
| 2b | 30 | 106 | 99 |
| | 100 | 114 | 105 |
| 2c | 10 | 103 | 102 |
| | 30 | 111 | 104 |
| 2d | 10 | 103 | 100 |
| | 30 | 111 | 104 |
| 2j | 100 | 110 | - |
| 2o | 30 | 108 | - |
| | 100 | 111 | - |
| 3a | 10 | 108 | - |
| | 30 | 115 | - |

### Beispiel 1

Eine Lösung von 15 g LiAlH₄ in 600 ml Diäthyläther wird unter Rühren mit einer Lösung von 20 g [[[p-(2-Aethoxyäthoxy)phenäthyl]carbamoyl]phenyl]essigsäureäthylester versetzt. Nach 12 Stunden Rühren werden dem Gemisch 15 ml Wasser und 5 ml 10% Natronlauge zugetropft. Das Gemisch wird durch Kieselgel filtriert und das Filtrat wird eingedampft. Der Rückstand wird in Aethanol gelöst und mit der in Aethanol gelösten äquivalenten Menge Oxalsäure versetzt. Nach Zugabe von Aethylacetat erhält man das β-[[[p-(2-Aethoxyäthoxy)-phenäthyl]amino]methyl]phenäthylalkohol-oxalat
Smp. 145°C.

### Beispiel 2

Analog Beispiel 1 wurden hergestellt:
2a) aus [[(R)-p-(2-Aethoxyäthoxy)-α-methylphenäthyl]carbamoyl]phenylessigsäureäthylester das β-[[[(R)-p-(2-Aethoxyäthoxy)-α-methylphenäthyl]amino]methyl]phenäthylalkohol-oxalat
   Smp. 145°C (Zersetzung).
2b) aus [[p-(2-Aethoxyäthoxy)phenäthyl]carbamoyl]-m-chlor-phenylessigsäureäthylester das m-Chlor-β-[[[p-(2-äthoxyäthoxy)phenäthyl]amino]methyl]phenäthylalkohol-oxalat
   Smp. 148°C.
2c) aus (RS)-2-Phenyl-N-[(RS)-β-(hydroxymethyl)phenäthyl]-N-[p-(2-äthoxyäthoxy)phenäthyl]äthylmalonamat das β,β′-[[[p-(2-Aethoxyäthoxy)phenäthyl]imino]dimethylen]bis-[(RS)-phenäthylalkohol]hydrochlorid
   Smp. 94°C.
2d) aus (RS)-2-(m-Chlorphenyl)-N-[p-(2-äthoxyäthoxy)-phenäthyl]-N-[(R)-β-hydroxyphenäthyl]malonamat das (RS)-m-Chlor-β-[p-(2-äthoxyäthoxy)phenäthyl][[[(R)-β-hydroxyphenäthyl]amino]methyl]phenäthylalkohol-oxalat
   Smp. 130°C.
2e) aus (RS)-[[[(R)-α-Methyl-p-(2-phenäthoxy)äthoxy]phenäthyl]carbamoyl]-m-chlorphenylessigsäureäthylester das (RS)-m-Chlor-β-[[[(R)-α-methyl-p-[2-(phenäthoxy)äthoxy]-phenäthyl]amino]methyl]phenäthylalkohol-oxalat
   Smp. 164-166°C.
2f) aus (RS)-2-(m-Chlorphenyl)-N-[(RS)-m-chlor-β-(hydroxymethyl)phenäthyl]-N-[p-(2-äthoxyäthoxy)phenäthyl]äthylmalonamat das β,β′-[[[p-(2-Aethoxyäthoxy)phenäthyl]imino]dimethylen]bis-[(RS)-m-chlorphenäthylalkohol]hydrochlorid
   Smp. 148°C.
2g) aus (RS)-[[[(R)-α-Methyl-p-(2-äthoxyäthoxy)phenäthyl]carbamoyl]-m-chlorphenylessigsäureäthylester das (RS)-m-Chlor-β-[[[(R)-p-(2-äthoxyäthoxy)-α-methylphenäthyl]amino]methyl]phenäthylalkohol-oxalat
   Smp. 145°C.
2h) aus [[p-Hydroxyphenäthyl]carbamoyl]phenylessigsäureäthylester das β-[[(p-Hydroxyphenäthyl)amino]methyl]phenäthylalkohol-oxalat
   Smp. 136-137°C.
2i) aus (RS)-[[[(R)-α-methyl-p-(2-phenäthoxy)äthoxy]phenäthyl]carbamoyl]phenylessigsäureäthylester das β-[[[(R)-α-Methyl-p-(2-phenäthoxy)äthoxy]phenäthyl]amino]methyl]phenäthylalkohol-oxalat
   Smp. 165-179°C.
2j) aus [[p-(2-Phenäthoxy)äthoxy]phenäthyl]carbamoyl]phenylessigsäureäthylester das β-[[[p-(2-Phenäthoxy)äthoxy]phenäthyl]amino]methyl]phenäthylalkohol-oxalat
   Smp. 145°C.
2k) aus [[p-(2-Aethoxyäthoxy)phenäthyl]carbamoyl]-m-trifluormethylphenylessigsäureäthylester das β-[[[p-(2-Aethoxyäthoxy)phenäthyl]amino]methyl]-m-trifluormethylphenäthylalkohol-oxalat
   Smp. 148°C.
2l) aus α-[[p-(2-Aethoxyäthoxy)phenäthyl]carbamoyl]-3-thiophenessigsäureäthylester das β-[[[p-(2-Aethoxyäthoxy)phenäthyl]amino]methyl]-3-thiophenäthanol-oxalat
   Smp. 153-155°C.
2m) aus α-[[p-(2-Aethoxyäthoxy)phenäthyl]carbamoyl]-2-pyridinessigsäureäthylester das β-[[[p-(2-Aethoxyäthoxy)phenäthyl]amino]methyl]-2-pyridinäthanol-oxalat
   Smp. 128-129°C.
2n) aus α-[[p-(2-Aethoxyäthoxy)phenäthyl]carbamoyl]-3-pyridinessigsäureäthylester das β-[[[p-(2-Aethoxyäthoxy)phenäthyl]amino]methyl]-3-pyridinäthanol-oxalat
   Smp. 170°C (Zersetzung).
2o) aus α-[[p-(2-Aethoxyäthoxy)phenäthyl]carbamoyl]-2-thiophenessigsäureäthylester das β-[[[p-(2-Aethoxyäthoxy)phenäthyl]amino]methyl]-2-thiophenäthanol-oxalat
   Smp. 145°C (Zersetzung).

### Beispiel 3

Analog Beispiel 1 werden hergestellt:
3a) aus N-[(R oder S)-3-Hydroxy-2-phenylpropyl]-p-(2-äthoxyäthoxy)phenylessigsäureamid das (R oder S)-β-[[[p-(2-Aethoxyäthoxy)phenäthyl]amino]methyl]phenäthylalkohol-oxalat
   Smp. 152-154°C, [α]_{D} -9° (DMSO, c = 1%)
3b) aus dem Beispiel 3a) entsprechenden Enantiomeren das Enantiomere (R oder S)-β-[[[p-(2-Aethoxyäthoxy)phenäthyl]amino]methyl]phenäthylalkohol-oxalat
   Smp. 151-154°C, [α]_{D} +8,3° (DMSO, c = 1%).

### Beispiel 4

3,62 g des Produkts von Beispiel 2h) werden in 200 ml Aceton mit 2,5 g Jodessigsäureäthylester und 2 g Kaliumhydroxyd 24 Stunden gerührt. Dann wird eingedampft, der Rückstand wird in Wasser und Essigester aufgenommen. Die organische Phase wird abgetrennt, über Natriumsulfat getrocknet und eingedampft. Der Rückstand wird an Kieselgel chromatographiert. Das Produkt wird in Aethanol gelöst, mit 1 ml konzentrierter Salzsäure versetzt und mit Aethanol abgedampft. Kristallisation des Rückstandes aus Aethanol/Aether ergibt [p-[2-[[β-(Hydroxymethyl)phenäthyl]amino]äthyl]phenoxy]essigsäureäthylester-hydrochlorid
Smp. 104-105°C.

### Beispiel 5

Es werden in üblicher Weise Tabletten folgender Zusammensetzung hergestellt:

| Wirkstoff der Formel I, z.B. | |
|---|---|
| (RS)-m-Chlor-ß-[p-(2-äthoxyäthoxy) phenäthyl][[[(R)-ß-hydroxyphenäthyl] amino]methyl]phenäthylalkohol-oxalat | 250 mg |
| Lactose | 200 mg |
| Maisstärke | 300 mg |
| Maisstärkepaste | 50 mg |
| Calciumstearat | 5 mg |
| Dicalciumphosphat | 45 mg |

## Patentansprüche (Patentansprüche für folgende(n) Vertragsstaat(en): AT, BE, CH, DE, FR, GB, IT, LI, LU, NL, SE)

1. Propanolaminderivate der Formel 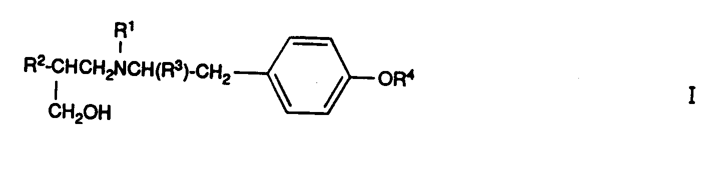 worin R¹ Wasserstoff oder eine Gruppe der Formel 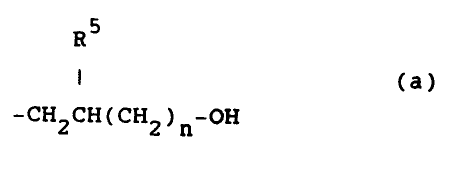
n die Zahl 0 oder 1
R² und R⁵ Phenyl, m-Halophenyl, m-Trifluormethylphenyl, Thienyl oder Pyridyl
R³ Wasserstoff oder Methyl
R⁴ Wasserstoff, -CH₂COOH, -CH₂COO-C₁₋₄-Alkyl, -(CH₂)₂O-C₁₋₄-Alkyl oder -(CH₂)₂O(CH₂)₁₋₄-C₆H₅ ist,
und physiologisch verträgliche Salze davon.

2. Verbindungen nach Anspruch 1, worin R¹ Wasserstoff ist.

3. Verbindungen nach Anspruch 1, worin R¹ eine Gruppe (a) darstellt, in der n und R⁵ die im Anspruch 1 angegebene Bedeutung haben.

4. Verbindungen nach Anspruch 1 oder 2, worin R¹ Wasserstoff, R² Phenyl, m-Halophenyl, insbesondere m-Chlorphenyloder m-Trifluormethylphenyl, Thienyl oder Pyridyl, insbesondere 2- oder 3-Pyridyl, R⁴ Wasserstoff, 2-Aethoxyäthyl, 2-Phenäthoxyäthyl oder Aethoxycarbonylmethyl ist, insbesondere diejenigen, worin das an einer allfälligen Methylgruppe R³ gebundene C-Atom die R-Konfiguration hat.

5. Verbindungen nach Anspruch 1 oder 3, worin R¹ eine Gruppe (a) darstellt, in der entweder n die Zahl 1 und R⁵ Phenyl oder m-Halophenyl, insbesondere m-Chlorphenyl, oder n die Zahl 0 und R⁵ Phenyl ist, insbesondere worin das an der Gruppe R⁵ gebundene C-Atom die R-Konfiguration hat, R² Phenyl oder m-Halophenyl, insbesondere m-Chlorphenyl, R³ Wasserstoff und R⁴ 2-Aethoxyäthyl ist.

6. Verbindungen nach Anspruch 4, worin R² Phenyl oder 2-Thienyl und R⁴ 2-Aethoxyäthyl oder Aethoxycarbonylmethyl ist.

7. Verbindungen nach Anspruch 5, worin R¹ β-Hydroxyphenäthyl, insbesondere (R)-β-Hydroxyphenäthyl, R² m-Chlorphenyl, R³ Wasserstoff und R⁴ 2-Aethoxyäthyl ist.

8. (RS)-m-Chlor-β-[p-(2-äthoxyäthoxy)phenäthyl][[[(R)-β-hydroxyphenäthyl]amino]methyl]phenäthylalkohol.

9. Eine Verbindung nach Anspruch 6 aus der Gruppe der folgenden
(R oder S)-β-[[[p-(2-Aethoxyäthoxy)phenäthyl]amino]-methyl]phenäthylalkohol,
[p-[2-[[β-(Hydroxymethyl)phenäthyl]amino]äthyl]phenoxy]-essigsäureäthylester,
β-[[[(R)-p-(2-Aethoxyäthoxy)-α-methylphenäthyl]amino]-methyl]phenäthylalkohol und
β-[[[p-(2-Aethoxyäthoxy)phenäthyl]amino]methyl]-2-thiophenäthanol.

10. Verbindungen nach einem der Ansprüche 1-9 als therapeutische Wirkstoffe, insbesondere zur Behandlung der Fettsucht, des Diabetes mellitus und von Zuständen, die mit erhöhtem Proteinabbau verbunden sind.

11. Pharmazeutische Präparate, gekennzeichnet durch einen Gehalt an einer Verbindung nach einem der Ansprüche 1-9.

12. Futtermittel für Masttiere enthaltend eine Verbindung nach einem der Ansprüche 1-9.

13. Verfahren zur Herstellung der Verbindungen nach einem der Ansprüche 1-9, dadurch gekennzeichnet, dass man
a) ein Amid der Formel 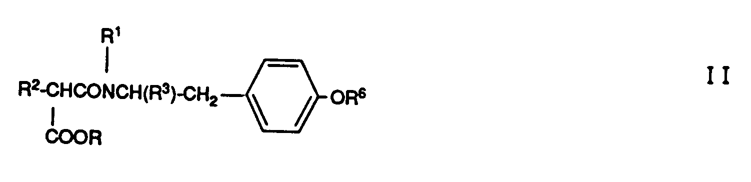 oder 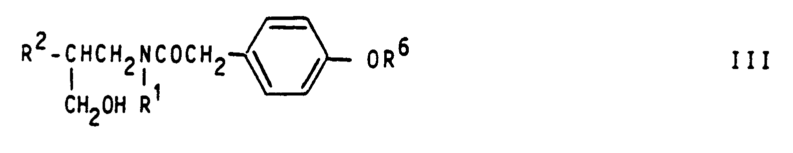 worin R C₁₋₄-Alkyl bedeutet und
worin R¹, R² oder R³ die im Anspruch 1 angegebene Bedeutung haben und R⁶ Wasserstoff, -(CH₂)₂O-C₁₋₄-Alkyl oder -(CH₂)₂O(CH₂)₁₋₄-C₆H₅ ist,
reduziert und
b) gewünschtenfalls ein Phenol der Formel I, worin R⁴ Wasserstoff ist, zu einer Verbindung der Formel I, worin R⁴ eine Gruppe -CH₂-COOH oder -CH₂COO-C₁₋₄-Alkyl ist,
veräthert und
c) gewünschtenfalls eine Verbindung der Formel I in ein Salz überführt.

14. Verwendung einer Verbindung nach einem der Ansprüche 1-9 zur Herstellung von pharmazeutischen Präparaten zur Behandlung der Fettsucht, des Diabetes mellitus und von Zuständen, die mit erhöhtem Proteinabbau verbunden sind.

## Patentansprüche (Patentansprüche für folgende(n) Vertragsstaat(en): ES)

1. Verfahren zur Herstellung von Propanolaminderivaten der Formel 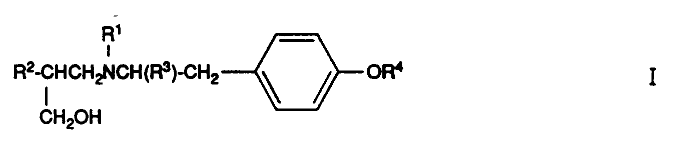 worin R¹ Wasserstoff oder eine Gruppe der Formel 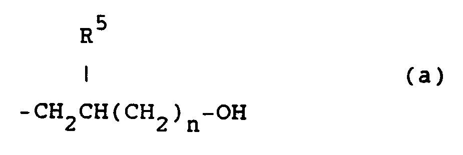
n die Zahl 0 oder 1
R² und R⁵ Phenyl, m-Halophenyl, m-Trifluormethylphenyl, Thienyl oder Pyridyl
R³ Wasserstoff oder Methyl
R⁴ Wasserstoff, -CH₂COOH, -CH₂COO-C₁₋₄-Alkyl, -(CH₂)₂O-C₁₋₄-Alkyl oder -(CH₂)₂O(CH₂)₁₋₄-C₆H₅ ist,
und physiologisch verträgliche Salze davon, dadurch gekennzeichnet, dass man
a) ein Amid der Formel 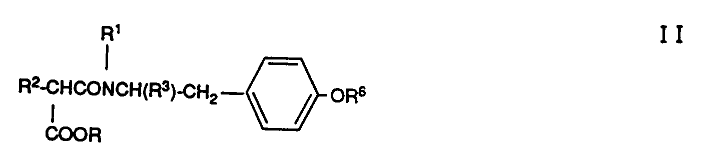 oder 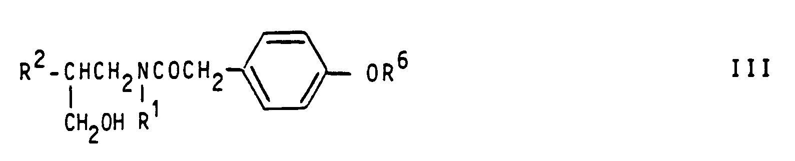 worin R C₁₋₄-Alkyl bedeutet und
worin R¹, R² oder R³ die obige Bedeutung haben und
R⁶ Wasserstoff, -(CH₂)₂O-C₁₋₄-Alkyl oder -(CH₂)₂O(CH₂)₁₋₄-C₆H₅ ist,
reduziert und
b) gewünschtenfalls ein Phenol der Formel I, worin R⁴ Wasserstoff ist, zu einer Verbindung der Formel I, worin R⁴ eine Gruppe -CH₂-COOH oder -CH₂COO-C₁₋₄-Alkyl ist, veräthert und
c) gewünschtenfalls eine Verbindung der Formel I in ein Salz überführt.

2. Verfahren nach Anspruch 1, worin R¹ Wasserstoff ist.

3. Verfahren nach Anspruch 1, worin R¹ eine Gruppe (a) darstellt, in der n und R⁵ die im Anspruch 1 angegebene Bedeutung haben.

4. Verfahren nach Anspruch 1 oder 2, worin R¹ Wasserstoff, R² Phenyl, m-Halophenyl, insbesondere m-Chlorphenyl oder m-Trifluormethylphenyl, Thienyl oder Pyridyl, insbesondere 2- oder 3-Pyridyl, R⁴ Wasserstoff, 2-Aethoxyäthyl, 2-Phenäthoxyäthyl oder Aethoxycarbonylmethyl ist, insbesondere diejenigen, worin das an einer allfälligen Methylgruppe R³ gebundene C-Atom die R-Konfiguration hat.

5. Verfahren nach Anspruch 1 oder 3, worin R¹ eine Gruppe (a) darstellt, in der entweder n die Zahl 1 und R⁵ Phenyl oder m-Halophenyl, insbesondere m-Chlorphenyl, oder n die Zahl 0 und R⁵ Phenyl ist, insbesondere worin das an der Gruppe R⁵ gebundene C-Atom die R-Konfiguration hat, R² Phenyl oder m-Halophenyl, insbesondere m-Chlorphenyl, R³ Wasserstoff und R⁴ 2-Aethoxyäthyl ist.

6. Verfahren nach Anspruch 4, worin R² Phenyl oder 2-Thienyl und R⁴ 2-Aethoxyäthyl oder Aethoxycarbonylmethyl ist.

7. Verfahren nach Anspruch 5, worin R¹ β-Hydroxyphenäthyl, insbesondere (R)-β-Hydroxyphenäthyl, R² m-Chlorphenyl, R³ Wasserstoff und R⁴ 2-Aethoxyäthyl ist.

8. Verfahren nach Anspruch 1, dadurch gekennzeichnet, dass man den (RS)-m-Chlor-β-[p-(2-äthoxyäthoxy)phenäthyl]-[[[(R)-β-hydroxyphenäthyl]amino]methyl]phenäthylalkohol herstellt.

9. Verfahren nach Anspruch 1, dadurch gekennzeichnet, dass man eine Verbindung aus der Gruppe der folgenden herstellt:
(R oder S)-β-[[[p-(2-Aethoxyäthoxy)phenäthyl]amino]methyl]phenäthylalkohol,
[p-[2-[[β-(Hydroxymethyl)phenäthyl]amino]äthyl]phenoxy]essigsäureäthylester,
β-[[[(R)-p-(2-Aethoxyäthoxy)-α-methylphenäthyl]amino]methyl]phenäthylalkohol und
β-[[[p-(2-Aethoxyäthoxy)phenäthyl]amino]methyl]-2-thiophenäthanol.

10. Verfahren zur Herstellung von Arzneimitteln, insbesondere zur Behandlung der Fettsucht, des Diabetes mellitus und von Zuständen, die mit erhöhtem Proteinabbau verbunden sind, dadurch gekennzeichnet, dass man eine Verbindung nach einem der Anprüche 1-9 in eine galenische Darreichungsform bringt.

11. Verwendung einer Verbindung nach einem der Ansprüche 1-9 zur Herstellung von pharmazeutischen Präparaten zur Behandlung der Fettsucht, des Diabetes mellitus und von Zuständen, die mit erhöhtem Proteinabbau verbunden sind.

12. Verfahren nach Anspruch 1, dadurch gekennzeichnet, dass man
a) das Amid der Formel II oder III mit einem komplexen Metallhydrid, in einem ätherischen Lösungsmittel, bei einer Temperatur bis zur Rückflusstemperatur reduziert und
b) gewünschtenfalls ein Phenol der Formel I, worin R⁴ Wasserstoff ist, mit einer Verbindung der Formel
X-R⁴⁰
worin R⁴⁰ eine Gruppe -CH₂COOH oder -CH₂COO-C₁₋₄-Alkyl und X Halogen oder eine Sulfonatgruppe ist,
in einem Lösungsmittel, in Gegenwart einer Base bei einer Temperatur bis zur Rückflusstemperatur veräthert.

## Patentansprüche (Patentansprüche für folgende(n) Vertragsstaat(en): GR)

1. Verfahren zur Herstellung von Propanolaminderivaten der Formel 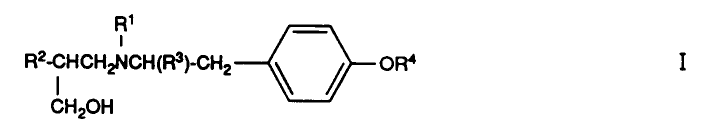 worin R¹ Wasserstoff oder eine Gruppe der Formel 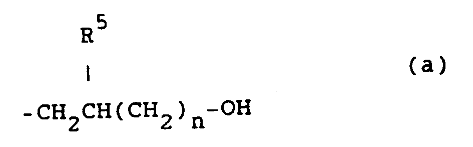
n die Zahl 0 oder 1
R² und R⁵ Phenyl, m-Halophenyl, m-Trifluormethylphenyl, Thienyl oder Pyridyl
R³ Wasserstoff oder Methyl
R⁴ Wasserstoff, -CH₂COOH, -CH₂COO-C₁₋₄-Alkyl, -(CH₂)₂O-C₁₋₄-Alkyl oder -(CH₂)₂O(CH₂)₁₋₄-C₆H₅ ist,
und physiologisch verträgliche Salze davon, dadurch gekennzeichnet, dass man
a) ein Amid der Formelsx 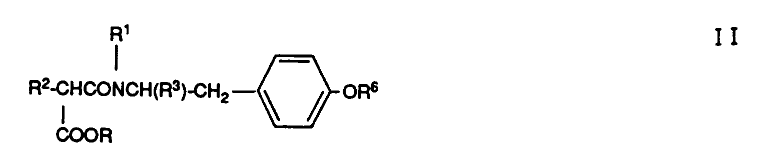 oder 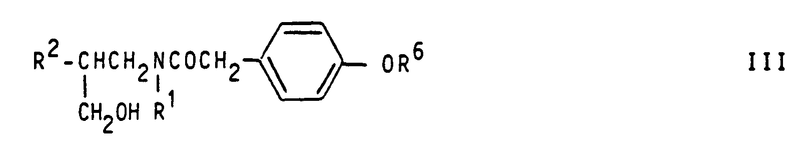 worin R C₁₋₄-Alkyl bedeutet und
worin R¹ R² oder R³ die obige Bedeutung haben und
R⁶ Wasserstoff, -(CH₂)₂O-C₁₋₄-Alkyl oder -(CH₂)₂O(CH₂)₁₋₄-C₆H₅ ist,
reduziert und
b) gewünschtenfalls ein Phenol der Formel I, worin R⁴ Wasserstoff ist, zu einer Verbindung der Formel I, worin R⁴ eine Gruppe -CH₂-COOH oder -CH₂COO-C₁₋₄-Alkyl ist, veräthert und
c) gewünschtenfalls eine Verbindung der Formel I in ein Salz überführt.

2. Verfahren nach Anspruch 1, worin R¹ Wasserstoff ist.

3. Verfahren nach Anspruch 1, worin R¹ eine Gruppe (a) darstellt, in der n und R⁵ die im Anspruch 1 angegebene Bedeutung haben.

4. Verfahren nach Anspruch 1 oder 2, worin R¹ Wasserstoff, R² Phenyl, m-Halophenyl, insbesondere m-Chlorphenyl oder m-Trifluormethylphenyl, Thienyl oder Pyridyl, insbesondere 2- oder 3-Pyridyl, R⁴ Wasserstoff, 2-Aethoxyäthyl, 2-Phenäthoxyäthyl oder Aethoxycarbonylmethyl ist, insbesondere diejenigen, worin das an einer allfälligen Methylgruppe R³ gebundene C-Atom die R-Konfiguration hat.

5. Verfahren nach Anspruch 1 oder 3, worin R¹ eine Gruppe (a) darstellt, in der entweder n die Zahl 1 und R⁵ Phenyl oder m-Halophenyl, insbesondere m-Chlorphenyl, oder n die Zahl 0 und R⁵ Phenyl ist, insbesondere worin das an der Gruppe R⁵ gebundene C-Atom die R-Konfiguration hat, R² Phenyl oder m-Halophenyl, insbesondere m-Chlorphenyl, R³ Wasserstoff und R⁴ 2-Aethoxyäthyl ist.

6. Verfahren nach Anspruch 4, worin R² Phenyl oder 2-Thienyl und R⁴ 2-Aethoxyäthyl oder Aethoxycarbonylmethyl ist.

7. Verfahren nach Anspruch 5, worin R¹ β-Hydroxyphenäthyl, insbesondere (R)-β-Hydroxyphenäthyl, R² m-Chlorphenyl, R³ Wasserstoff und R⁴ 2-Aethoxyäthyl ist.

8. Verfahren nach Anspruch 1, dadurch gekennzeichnet, dass man den (RS)-m-Chlor-β-[p-(2-äthoxyäthoxy)phenäthyl][[[(R)-β-hydroxyphenäthyl]amino]methyl]phenäthylalkohol herstellt.

9. Verfahren nach Anspruch 1, dadurch gekennzeichnet, dass man eine Verbindung aus der Gruppe der folgenden herstellt:
(R oder S)-β-[[[p-(2-Aethoxyäthoxy)phenäthyl]amino]methyl]phenäthylalkohol,
[p-[2-[ [β-(Hydroxymethyl)phenäthyl]amino]äthyl]phenoxy]-essigsäureäthylester,
β-[[[(R)-p-(2-Aethoxyäthoxy)-α-methylphenäthyl]amino]methyl]phenäthylalkohol und
β-[[[p-(2-Aethoxyäthoxy)phenäthyl]amino]methyl]-2-thiophenäthanol.

10. Verfahren zur Herstellung von Arzneimitteln, insbesondere zur Behandlung der Fettsucht, des Diabetes mellitus und von Zuständen, die mit erhöhtem Proteinabbau verbunden sind, dadurch gekennzeichnet, dass man eine Verbindung nach einem der Anprüche 1-9 in eine galenische Darreichungsform bringt.

11. Verwendung einer Verbindung nach einem der Ansprüche 1-9 zur Herstellung von pharmazeutischen Präparaten zur Behandlung der Fettsucht, des Diabetes mellitus und von Zuständen, die mit erhöhtem Proteinabbau verbunden sind.

## Claims (Claims for the following Contracting State(s): AT, BE, CH, DE, FR, GB, IT, LI, LU, NL, SE)

1. Propanolamine derivatives of the formula 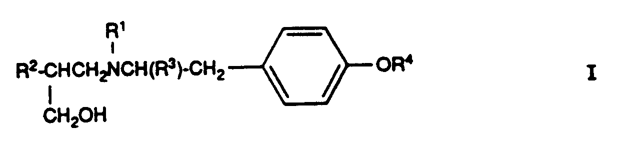 wherein
R¹ is hydrogen or a group of the formula 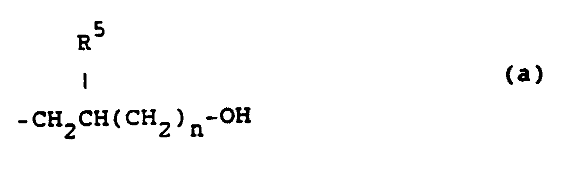
n is the number 0 or 1
R² and R⁵ are phenyl, m-halophenyl, m-trifluoromethylphenyl, thienyl or pyridyl
R³ is hydrogen or methyl
R⁴ is hydrogen, -CH₂COOH, -CH₂COO-C₁₋₄-alkyl, -(CH₂)₂O-C₁₋₄-alkyl or -(CH₂)₂O(CH₂)₁₋₄-C₆H₅,
and physiologically compatible salts thereof.

2. Compounds according to claim 1, wherein R¹ is hydrogen.

3. Compounds according to claim 1, wherein R¹ represents a group (a) in which n and R⁵ have the significance given in claim 1.

4. Compounds according to claim 1 or 2, wherein R¹ is hydrogen, R² is phenyl, m-halophenyl, especially m-chlorophenyl, or m-trifluoromethylphenyl, thienyl or pyridyl, especially 2- or 3-pyridyl, R⁴ is hydrogen, 2-ethoxyethyl, 2-phenethoxyethyl or ethoxycarbonylmethyl, especially those in which the C-atom attached to a methyl group R³ which may be present has the R-configuration.

5. Compounds according to claim 1 or 3, wherein R¹ represents a group (a) in which either n is the number 1 and R⁵ is phenyl or m-halophenyl, especially m-chlorophenyl, or n is the number 0 and R⁵ is phenyl, especially in which the C-atom attached to the group R⁵ has the R-configuration, R² is phenyl or m-halophenyl, especially m-chlorophenyl, R³ is hydrogen and R⁴ is 2-ethoxyethyl.

6. Compounds according to claim 4, wherein R² is phenyl or 2-thienyl and R⁴ is 2-ethoxyethyl or ethoxycarbonylmethyl.

7. Compounds according to claim 5, wherein R¹ is β-hydroxyphenethyl, especially (R)-β-hydroxyphenethyl, R² is m-chlorophenyl, R³ is hydrogen and R⁴ is 2-ethoxyethyl.

8. (RS)-m-Chloro-β-[p-(2-ethoxyethoxy)phenethyl] [[[(R)-β-hydroxyphenethyl]amino]methyl]phenethyl alcohol.

9. A compound according to claim 6 from the following group
(R or S)-β-[[[p-(2-ethoxyethoxy)phenethyl]amino]methyl]phenethyl alcohol,
ethyl [p-[2-[[β-(hydroxymethyl)phenethyl]amino]ethyl]phenoxy]acetate,
β-[[[(R)-p-(2-ethoxyethoxy)-α-methylphenethyl]amino]methyl]phenethyl alcohol and
β-[[[p-(2-ethoxyethoxy)phenethyl]amino]methyl]-2-thiopheneethanol.

10. Compounds according to any one of claims 1-9 as therapeutically active substances, especially for the treatment of obesity, of diabetes mellitus and of conditions which are associated with high protein breakdown.

11. Pharmaceutical preparations, characterized by a content of a compound according to any one of claims 1-9.

12. Feedstuff for fattening animals containing a compound according to any one of claims 1-9.

13. A process for the manufacture of the compounds according to any one of claims 1-9, characterized by
a) reducing an amide of the formula 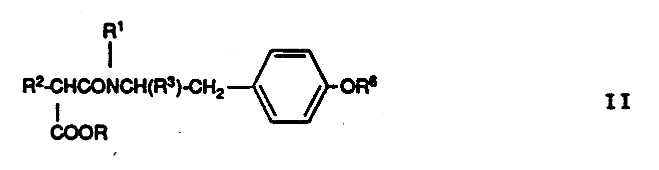 or 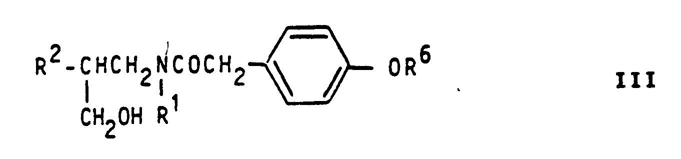 wherein R signifies C₁₋₄-alkyl and wherein R¹, R² or R³ have the significance given in claim 1 and R⁶ is hydrogen, -(CH₂)₂O-C₁₋₄-alkyl or -(CH₂)₂O(CH₂)₁₋₄-C₆H₅,
and
b) if desired, etherifying a phenol of formula I in which R⁴ is hydrogen to a compound of formula I in which R⁴ is a group -CH₂-COOH or -CH₂COO-C₁₋₄-alkyl, and
c) if desired, converting a compound of formula I into a salt.

14. The use of a compound according to any one of claims 1-9 for the manufacture of pharmaceutical preparations for the treatment of obesity, of diabetes mellitus and of conditions which are associated with high protein breakdown.

## Claims (Claims for the following Contracting State(s): ES)

1. A process for the manufacture of propanolamine derivatives of the formula 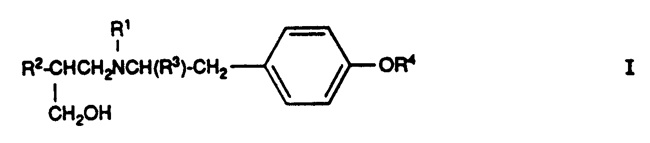 wherein
R¹ is hydrogen or a group of the formula 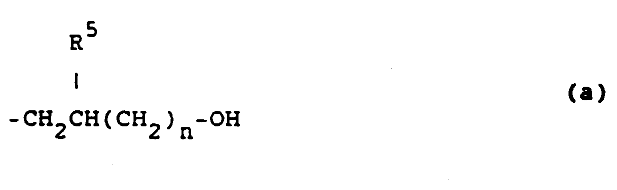
n is the number 0 or 1
R² and R⁵ are phenyl, m-halophenyl, m-trifluoromethylphenyl, thienyl or pyridyl
R³ is hydrogen or methyl
R⁴ is hydrogen, -CH₂COOH, -CH₂COO-C₁₋₄-alkyl, -(CH₂)₂O-C₁₋₄-alkyl or -(CH₂)₂O(CH₂)₁₋₄-C₆H₅,
and physiologically compatible salts thereof, characterized by
a) reducing an amide of the formula 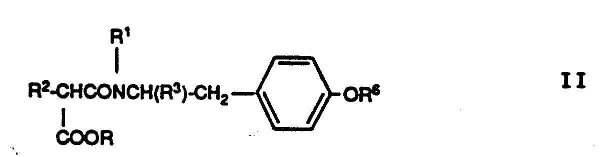 or 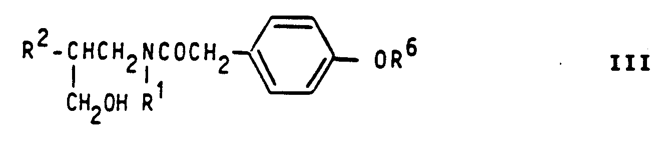 wherein R signifies C₁₋₄-alkyl and wherein R¹, R² or R³ have the above significance and R⁶ is hydrogen, -(CH₂)₂O-C₁₋₄-alkyl or -(CH₂)₂O(CH₂)₁₋₄-C₆H₅,
and
b) if desired, etherifying a phenol of formula I in which R⁴ is hydrogen to a compound of formula I in which R⁴ is a group -CH₂-COOH or -CH₂COO-C₁₋₄-alkyl, and
c) if desired, converting a compound of formula I into a salt.

2. A process according to claim 1, wherein R¹ is hydrogen.

3. A process according to claim 1, wherein R¹ represents a group (a) in which n and R⁵ have the significance given in claim 1.

4. A process according to claim 1 or 2, wherein R¹ is hydrogen, R² is phenyl, m-halophenyl, especially m-chlorophenyl, or m-trifluoromethylphenyl, thienyl or pyridyl, especially 2- or 3-pyridyl, R⁴ is hydrogen, 2-ethoxyethyl, 2-phenethoxyethyl or ethoxycarbonylmethyl, especially those in which the C-atom attached to a methyl group R³ which may be present has the R-configuration.

5. A process according to claim 1 or 3, wherein R¹ represents a group (a) in which either n is the number 1 and R⁵ is phenyl or m-halophenyl, especially m-chlorophenyl, or n is the number 0 and R⁵ is phenyl, especially in which the C-atom attached to the group R⁵ has the R-configuration, R² is phenyl or m-halophenyl, especially m-chlorophenyl, R³ is hydrogen and R⁴ is 2-ethoxyethyl.

6. A process according to claim 4, wherein R² is phenyl or 2-thienyl and R⁴ is 2-ethoxyethyl or ethoxycarbonylmethyl.

7. A process according to claim 5, wherein R¹ is β-hydroxyphenethyl, especially (R)-β-hydroxyphenethyl, R² is m-chlorophenyl, R³ is hydrogen and R⁴ is 2-ethoxyethyl.

8. A process according to claim 1, characterized in that (RS)-m-chloro-β-[p-(2-ethoxyethoxy)phenethyl] [[[(R)-β-hydroxyphenethyl]amino]methyl]phenethyl alcohol is manufactured.

9. A process according to claim 1, characterized in that a compound from the following group is manufactured:
(R or S)-β-[[[p-(2-ethoxyethoxy)phenethyl]amino]methyl]phenethyl alcohol,
ethyl [p-[2-[[β-(hydroxymethyl)phenethyl]amino]ethyl]phenoxy]acetate,
β-[[[(R)-p-(2-ethoxyethoxy)-α-methylphenethyl]amino]methyl]phenethyl alcohol and
β-[[[p-(2-ethoxyethoxy)phenethyl]amino]methyl]-2-thiopheneethanol.

10. A process for the manufacture of medicaments, especially for the treatment of obesity, of diabetes mellitus and of conditions which are associated with high protein breakdown, characterized by bringing a compound according to any one of claims 1-9 into a galenical administration form.

11. The use of a compound according to any one of claims 1-9 for the manufacture of pharmaceutical preparations for the treatment of obesity, of diabetes mellitus and of conditions which are associated with high protein breakdown.

12. A process according to claim 1, characterized in that
a) the amide of formula II or III is reduced with a complex metal hydride in an ethereal solvent at a temperature up to the reflux temperature and
b) if desired, a phenol of formula I in which R⁴ is hydrogen is etherified with a compound of the formula
X-R⁴⁰
wherein R⁴⁰ is a group -CH₂COOH or -CH₂COO-C₁₋₄-alkyl and X is halogen or a sulphonate group,
in a solvent in the presence of a base at a temperature up to the reflux temperature.

## Claims (Claims for the following Contracting State(s): GR)

1. A process for the manufacture of propanolamine derivatives of the formula 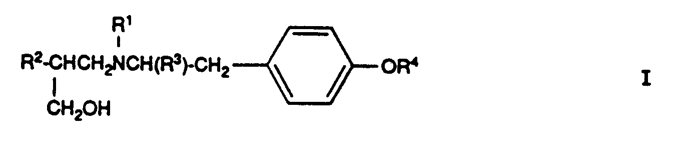 wherein
R¹ is hydrogen or a group of the formula 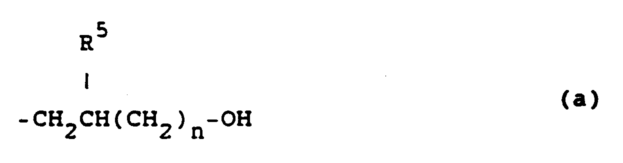
n is the number 0 or 1
R² and R⁵ are phenyl, m-halophenyl, m-trifluoromethylphenyl, thienyl or pyridyl
R³ is hydrogen or methyl
R⁴ is hydrogen, -CH₂COOH, -CH₂COO-C₁₋₄-alkyl, -(CH₂)₂O-C₁₋₄-alkyl or -(CH₂)₂O(CH₂)₁₋₄-C₆H₅,
and physiologically compatible salts thereof, characterized by
a) reducing an amide of the formula 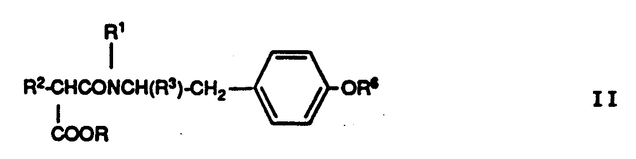 or 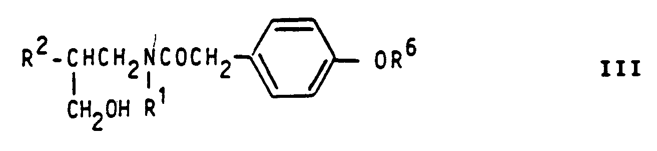 wherein R signifies C₁₋₄-alkyl and wherein R¹, R² or R³ have the above significance and R⁶ is hydrogen, -(CH₂)₂O-C₁₋₄-alkyl or -(CH₂)₂O(CH₂)₁₋₄-C₆H₅,
and
b) if desired, etherifying a phenol of formula I in which R⁴ is hydrogen to a compound of formula I in which R⁴ is a group -CH₂-COOH or -CH₂COO-C₁₋₄-alkyl, and
c) if desired, converting a compound of formula I into a salt.

2. A process according to claim 1, wherein R¹ is hydrogen.

3. A process according to claim 1, wherein R¹ represents a group (a) in which n and R⁵ have the significance given in claim 1.

4. A process according to claim 1 or 2, wherein R¹ is hydrogen, R² is phenyl, m-halophenyl, especially m-chlorophenyl, or m-trifluoromethylphenyl, thienyl or pyridyl, especially 2- or 3-pyridyl, R⁴ is hydrogen, 2-ethoxyethyl, 2-phenethoxyethyl or ethoxycarbonylmethyl, especially those in which the C-atom attached to a methyl group R³ which may be present has the R-configuration.

5. A process according to claim 1 or 3, wherein R¹ represents a group (a) in which either n is the number 1 and R⁵ is phenyl or m-halophenyl, especially m-chlorophenyl, or n is the number 0 and R⁵ is phenyl, especially in which the C-atom attached to the group R⁵ has the R-configuration, R² is phenyl or m-halophenyl, especially m-chlorophenyl, R³ is hydrogen and R⁴ is 2-ethoxyethyl.

6. A process according to claim 4, wherein R² is phenyl or 2-thienyl and R⁴ is 2-ethoxyethyl or ethoxycarbonylmethyl.

7. A process according to claim 5, wherein R¹ is β-hydroxyphenethyl, especially (R)-β-hydroxyphenethyl, R² is m-chlorophenyl, R³ is hydrogen and R⁴ is 2-ethoxyethyl.

8. A process according to claim 1, characterized in that (RS)-m-chloro-β-[p-(2-ethoxyethoxy)phenethyl] [[[(R)-β-hydroxyphenethyl]amino]methyl]phenethyl alcohol is manufactured.

9. A process according to claim 1, characterized in that a compound from the following group is manufactured:
(R or S)-β-[[[p-(2-ethoxyethoxy)phenethyl]amino]methyl]phenethyl alcohol,
ethyl [p-[2-[[β-(hydroxymethyl)phenethyl]amino]ethyl]phenoxy]acetate,
β-[[[(R)-p-(2-ethoxyethoxy)-α-methylphenethyl]amino]methyl]phenethyl alcohol and
β-[[[p-(2-ethoxyethoxy)phenethyl]amino]methyl]-2-thiopheneethanol.

10. A process for the manufacture of medicaments, especially for the treatment of obesity, of diabetes mellitus and of conditions which are associated with high protein breakdown, characterized by bringing a compound according to any one of claims 1-9 into a galenical administration form.

11. The use of a compound according to any one of claims 1-9 for the manufacture of pharmaceutical preparations for the treatment of obesity, of diabetes mellitus and of conditions which are associated with high protein breakdown.

## Revendications (Revendications pour l'(les) Etat(s) contractant(s) suivant(s): AT, BE, CH, DE, FR, GB, IT, LI, LU, NL, SE)

1. Dérivés de la propanolamine, de formule 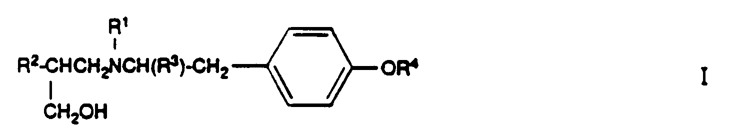 dans laquelle R¹ représente l'hydrogène ou un groupe de formule 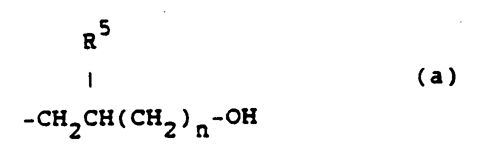
n est égal à 0 ou 1,
R² et R⁵ représentent des groupes phényle, m-halogénophényle, m-trifluorométhylphényle, thiényle ou pyridyle,
R³ représente l'hydrogène ou un groupe méthyle,
R⁴ représente l'hydrogène, -CH₂COOH, -CH₂COO-alkyle en C 1-C 4, -(CH₂)₂O-alkyle en C 1-C 4 ou -(CH₂)₂O(CH₂)_{1à4}-C₆H₅,
et leurs sels acceptables pour l'usage pharmaceutique.

2. Composés selon la revendication 1, pour lesquels R¹ représente l'hydrogène.

3. Composés selon revendication 1, pour lesquels R¹ représente un groupe (a) dans lequel n et R⁵ ont les significations indiquées dans la revendication 1.

4. Composés selon revendication 1 ou 2, pour lesquels R¹ représente l'hydrogène, R² un groupe phényle, m-halogénophényle, en particulier m-chlorophényle ou m-trifluorométhylphényle, thiényle ou pyridyle, en particulier 2- ou 3-pyridyle, R⁴ représente l'hydrogène, un groupe 2-éthoxyéthyle, 2-phénéthoxyéthyle ou éthoxycarbonylméthyle, en particulier ceux pour lesquels l'atome de carbone relié à un groupe méthyle R³ éventuel est en configuration R.

5. Composés selon la revendication 1 ou 3, pour lesquels R¹ représente un groupe (a) dans lequel n est égal à 1, et R⁵ représente un groupe phényle ou m-halogénophényle, en particulier m-chlorophényle ou bien n est égal à 0 et R⁵ représente un groupe phényle, en particulier ceux pour lesquels l'atome de carbone relié au groupe R⁵ est en configuration R, R² représente un groupe phényle ou m-halogénophényle, en particulier m-chlorophényle, R³ représente l'hydrogène et R⁴ un groupe éthoxyéthyle.

6. Composés selon revendication 4, pour lesquels R² représente un groupe phényle ou 2-thiényle et R⁴ représente un groupe 2-éthoxyéthyle ou éthoxycarbonylméthyle.

7. Composés selon revendication 5, pour lesquels R¹ représente un groupe bêta-hydroxyphénéthyle, en particulier (R)-bêta-hydroxyphénéthyle, R² représente un groupe m-chlorophényle, R³ l'hydrogène et R⁴ un groupe 2-éthoxyéthyle.

8. L'alcool (RS)-m-chloro-bêta-[p-(2-éthoxyéthoxy)-phénéthyl]-[[[(R)-bêta-hydroxyphénéthyl]-amino]-méthyl]-phénéthylique.

9. Un composé selon revendication 6 pris parmi les suivants :
l'alcool (R ou S)-bêta-[[[p-(2-éthoxyéthoxy)-phénéthyl]-amino]-méthyl]-phénéthylique,
le [p-[2-[[bêta-hydroxyméthyl)-phénéthyl]-amino]-éthyl]-phénoxy]-acétate d'éthyle,
l'alcool bêta-[[[-R)-p-(2-éthoxyéthoxy)-alpha-méthyl-phénéthyl]-amino]-méthyl]-phénéthylique, et
le bêta-[[[p-(2-éthoxyéthoxy)-phénéthyl]-amino]-méthyl]-2-thiophénéthanol.

10. Composés selon une des revendications 1 à 9, en tant que substances actives thérapeutiques, en particulier pour le traitement de l'obésité, du diabète sucré et des états en relation avec une dégradation accrue des protéines.

11. Compositions pharmaceutiques, caractérisées en ce qu'elles contiennent un composé selon l'une des revendications 1 à 9.

12. Aliment pour animaux à l'engrais, contenant un composé selon l'une des revendications 1 à 9.

13. Procédé de préparation des composés selon l'une des revendications 1 à 9, caractérisé en ce que :
a) on réduit un amide de formule 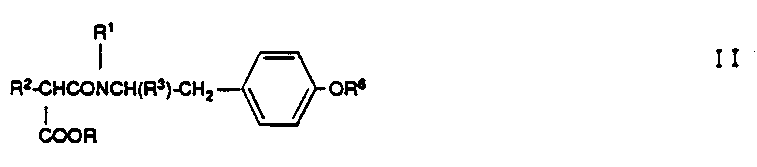 ou 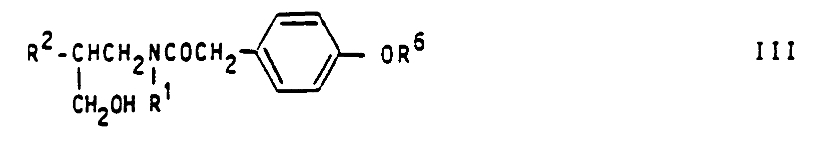 dans laquelle R représente un groupe alkyle en C 1-C 4 et
R¹, R² ou R³ ont les significations indiquées dans la revendication 1 et R⁶ représente l'hydrogène, un groupe (CH₂)₂O-alkyle en C 1-C 4 ou (CH₂)₂O(CH₂)_{1à4}-C₆H₅,
et
b) si on le désire, on éthérifie un phénol de formule I dans laquelle R⁴ représente l'hydrogène en un composé de formule I dans laquelle R⁴ représente un groupe -CH₂COOH ou -CH₂COO-alkyle en C 1-C 4, et
c) si on le désire, on convertit un composé de formule I en un sel.

14. Utilisation d'un composé selon une des revendications 1 à 9 pour la préparation de compositions pharmaceutiques prévues pour le traitement de l'obésité, du diabète sucré et des états en relation avec une dégradation accrue des protéines.

## Revendications (Revendications pour l'(les) Etat(s) contractant(s) suivant(s): ES)

1. Procédé de préparation de dérivés de la propanolamine de formule 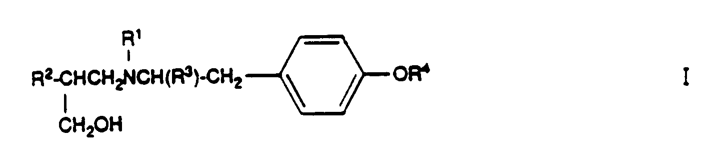 dans laquelle R¹ représente l'hydrogène ou un groupe de formule 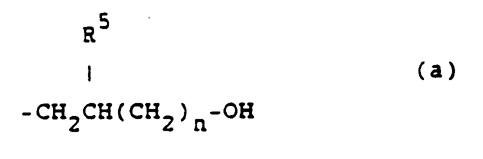
n est égal à 0 ou 1,
R² et R⁵ représentent des groupes phényle, m-halogénophényle, m-trifluorométhylphényle, thiényle ou pyridyle,
R³ représente l'hydrogène ou un groupe méthyle,
R⁴ représente l'hydrogène, un groupe -CH₂COOH, -CH₂COO-alkyle en C 1-C 4, -(CH₂)₂O)alkyle en C 1-C 4 ou -(CH₂)₂O(CH₂)_{1 à 4}-C₆H₅,
et de leurs sels acceptables pour l'usage pharmaceutique, caractérisé en ce que
a) on réduit un amide de formule 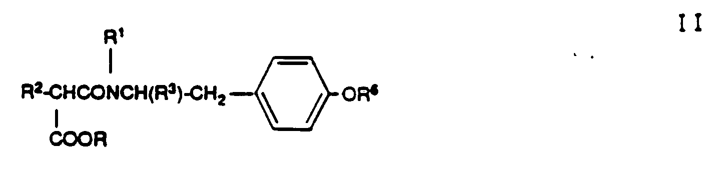 ou 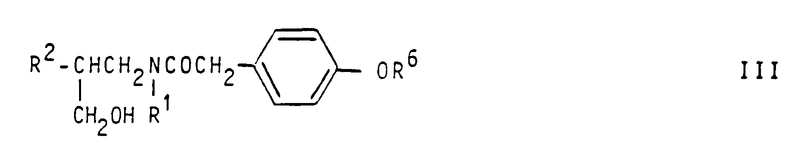 dans lesquelles R représente un groupe alkyle en C 1-C 4, et
R¹, R² ou R³ ont les significations indiquées ci-dessus et R⁶ représente l'hydrogène, un groupe -(CH₂)₂O-alkyle en C 1-C 4 ou (CH₂)₂O(CH₂)_{1 à 4}-C₆H₅, et
b) si on le désire, on éthérifie un phénol de formule I dans laquelle R⁴ représente l'hydrogène en un composé de formule I dans laquelle R⁴ représente un groupe -CH₂-COOH ou -CH₂COO-alkyle en C 1-C 4, et
c) si on le désire, on convertit un composé de formule I en un sel.

2. Procédé selon la revendication 1, dans laquelle R¹ représente l'hydrogène.

3. Procédé selon la revendication 1, dans laquelle R¹ représente un groupe (a) dans lequel n et R⁵ ont les significations indiquées dans la revendication 1.

4. Procédé selon la revendication 1 ou 2, dans lequel R¹ représente l'hydrogène, R² un groupe phényle, m-halogénophényle, en particulier m-chlorophényle ou m-trifluorométhylphényle, thiényle ou pyridyle, en particulier 2- ou 3-pyridyle, R⁴ représente l'hydrogène, un groupe 2-éthoxyéthyle, 2-phénéthoxyéthyle ou éthoxycarbonylméthyle, en particulier dans lequel l'atome de carbone relié à un groupe méthyle R³ éventuel est en configuration R.

5. Procédé selon la revendication 1 ou 3, dans lequel R¹ représente un groupe (a) dans lequel n est égal à 1 et R⁵ représente un groupe phényle ou m-halogénophényle, en particulier m-chlorophényle, ou bien n est égal à 0 et R⁵ représente un groupe phényle, dans lequel en particulier l'atome de carbone relié au groupe R⁵ est en configuration R, R² représente un groupe phényle ou m-halogénophényle, en particulier m-chlorophényle, R³ représente l'hydrogène et R⁴ un groupe 2-éthoxyéthyle.

6. Procédé selon revendication 4, dans lequel R² représente un groupe phényle ou 2-thiényle et R⁴ un groupe 2-éthoxyéthyle ou éthoxycarbonylméthyle.

7. Procédé selon la revendication 5, dans lequel R¹ représente un groupe bêta-hydroxyphénéthyle, en particulier (R)-bêta-hydroxyphénéthyle, R² représente un groupe m-chlorophényle, R³ l'hydrogène et R⁴ un groupe 2-éthoxyéthyle.

8. Procédé selon la revendication 1, caractérisé en ce que l'on prépare l'alcool (RS)-m-chlorobêta-[p-(2-éthoxyéthoxy)-phénéthyl]-[[[(R)-bêta-hydroxyphénéthyl]-amino]-méthyl]-phénéthylique.

9. Procédé selon revendication 1, caractérisé en ce que l'on prépare un composé pris parmi les suivants :
l'alcool (R ou S)-bêta-[[[p-(2-éthoxyéthoxy)-phénéthyl]-amino]-méthyl]-phénéthylique,
le [p-[2-[[bêta-(hydroxyméthyl)-phénéthyl]-amino]-éthyl]-phénoxy]-acétate d'éthyle,
l'alcool bêta-[[[(R)-p-(2-éthoxyéthoxy)-alpha-méthylphénéthyl]-amino]-méthyl]-phénéthylique, et
le bêta-[[[p-(2-éthoxyéthoxy)-phénéthyl]-amino]-méthyl]-2-thiophénéthanol.

10. Procédé de préparation des médicaments, en particulier pour le traitement de l'obésité, du diabète sucré et des états en relation avec une dégradation accrue des protéines, caractérisé en de que l'on met un composé selon l'une des revendications 1 à 9 sous une forme d'administration galénique.

11. Utilisation d'un composé selon l'une des revendications 1 à 9 pour la préparation de compositions pharmaceutiques pour le traitement de l'obésité, du diabète sucré et des états en relation avec une dégradation accrue des protéines.

12. Procédé selon la revendication 1, caractérisé en ce que :
a) on réduit l'amide de formule II ou III à l'aide d'un hydrure métallique complexe dans un solvant consistant en un éther à une température allant jusqu'à la température de reflux, et
b) si on le désire, on éthérifie un phénol de formule I dans laquelle R⁴ représente l'hydrogène à l'aide d'un composé de formule
X-R⁴⁰
dans laquelle R⁴⁰ représente un groupe -CH₂COOH ou -CH₂COO-alkyle en C 1-C 4, et X représente un halogène ou un groupe sulfonate,
dans un solvant, en présence d'une base, à une température allant jusqu'à la température de reflux.

## Revendications (Revendications pour l'(les) Etat(s) contractant(s) suivant(s): GR)

1. Procédé de préparation de dérivés de la propanolamine de formule 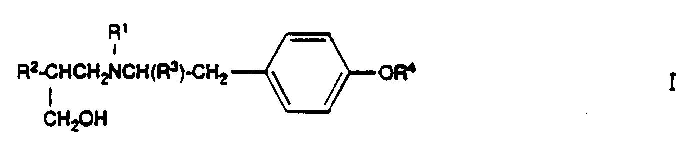 dans laquelle R¹ représente l'hydrogène ou un groupe de formule 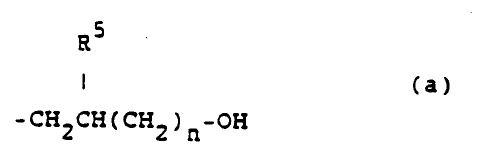
n est égal à 0 ou 1,
R² et R⁵ représentent des groupes phényle, m-halogénophényle, m-trifluorométhylphényle, thiényle ou pyridyle,
R³ représente l'hydrogène ou un groupe méthyle,
R⁴ représente l'hydrogène, un groupe -CH₂COOH, -CH₂COO-alkyle en C 1-C 4, -(CH₂)₂O)alkyle en C 1-C 4 ou -(CH₂)₂O(CH₂)_{1 à 4}-C₆H₅,
et de leurs sels acceptables pour l'usage pharmaceutique, caractérisé en ce que
a) on réduit un amide de formule 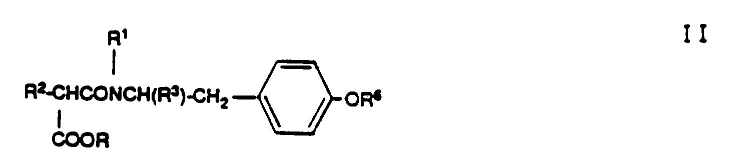 ou 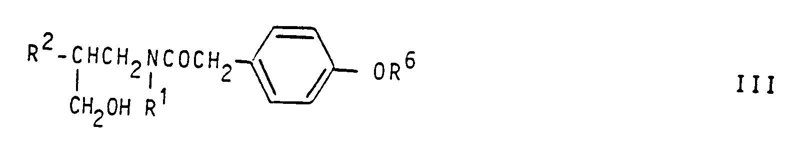 dans lesquelles R représente un groupe alkyle en C 1-C 4, et
R¹, R² ou R³ ont les significations indiquées ci-dessus et R⁶ représente l'hydrogène, un groupe -(CH₂)₂O-alkyle en C 1-C 4 ou (CH₂)₂O(CH₂)_{1 à 4}-C₆H₅, et
b) si on le désire, on éthérifie un phénol de formule I dans laquelle R⁴ représente l'hydrogène en un composé de formule I dans laquelle R⁴ représente un groupe -CH₂-COOH ou -CH₂COO-alkyle en C 1-C 4, et
c) si on le désire, on convertit un composé de formule I en un sel.

2. Procédé selon la revendication 1, dans laquelle R¹ représente l'hydrogène.

3. Procédé selon la revendication 1, dans laquelle R¹ représente un groupe (a) dans lequel n et R⁵ ont les significations indiquées dans la revendication 1.

4. Procédé selon la revendication 1 ou 2, dans lequel R¹ représente l'hydrogène, R² un groupe phényle, m-halogénophényle, en particulier m-chlorophényle ou m-trifluorométhylphényle, thiényle ou pyridyle, en particulier 2- ou 3-pyridyle, R⁴ représente l'hydrogène, un groupe 2-éthoxyéthyle, 2-phénéthoxyéthyle ou éthoxycarbonylméthyle, en particulier dans lequel l'atome de carbone relié à un groupe méthyle R³ éventuel est en configuration R.

5. Procédé selon la revendication 1 ou 3, dans lequel R¹ représente un groupe (a) dans lequel n est égal à 1 et R⁵ représente un groupe phényle ou m-halogénophényle, en particulier m-chlorophényle, ou bien n est égal à 0 et R⁵ représente un groupe phényle, dans lequel en particulier l'atome de carbone relié au groupe R⁵ est en configuration R, R² représente un groupe phényle ou m-halogénophényle, en particulier m-chlorophényle, R³ représente l'hydrogène et R⁴ un groupe 2-éthoxyéthyle.

6. Procédé selon revendication 4, dans lequel R² représente un groupe phényle ou 2-thiényle et R⁴ un groupe 2-éthoxyéthyle ou éthoxycarbonylméthyle.

7. Procédé selon la revendication 5, dans lequel R¹ représente un groupe bêta-hydroxyphénéthyle, en particulier (R)-bêta-hydroxyphénéthyle, R² représente un groupe m-chlorophényle, R³ l'hydrogène et R⁴ un groupe 2-éthoxyéthyle.

8. Procédé selon la revendication 1, caractérisé en ce que l'on prépare l'alcool (RS)-m-chloro-bêta-[p-(2-éthoxyéthoxy)-phénéthyl]-[[[(R)-bêta-hydroxyphénéthyl]-amino]-méthyl]-phénéthylique.

9. Procédé selon revendication 1, caractérisé en ce que l'on prépare un composé pris parmi les suivants :
l'alcool (R ou S)-bêta-[[[p-(2-éthoxyéthoxy)-phénéthyl]-amino]-méthyl]-phénéthylique,
le [p-[2-[[bêta-(hydroxyméthyl)-phénéthyl]-amino]-éthyl]-phénoxy]-acétate d'éthyle,
l'alcool bêta-[[[(R)-p-(2-éthoxyéthoxy)-alpha-méthylphénéthyl]-amino]-méthyl]-phénéthylique, et
le bêta-[[[p-(2-éthoxyéthoxy)-phénéthyl]-amino]-méthyl]-2-thiophénéthanol.

10. Procédé de préparation des médicaments, en particulier pour le traitement de l'obésité, du diabète sucré et des états en relation avec une dégradation accrue des protéines, caractérisé en ce que l'on met un composé selon l'une des revendications 1 à 9 sous une forme d'administration galénique.

11. Utilisation d'un composé selon l'une des revendications 1 à 9 pour la préparation de compositions pharmaceutiques pour le traitement de l'obésité, du diabète sucré et des états en relation avec une dégradation accrue des protéines.
